(19) Europäisches Patentamt
European Patent Office
Office européen des brevets

(11) Publication number: **0 023 751**
**B1**

(12) **EUROPEAN PATENT SPECIFICATION**

(45) Date of publication of patent specification: **14.12.83**

(21) Application number: **80301559.3**

(22) Date of filing: **13.05.80**

(51) Int. Cl.³: **C 07 D 207/267,**
C 07 D 223/10,
C 07 D 201/08,
C 07 D 211/76,
C 07 D 227/087,
B 01 J 23/46

(54) **Process for producing five, six or seven membered saturated nitrogen containing heterocyclic compounds.**

(30) Priority: **29.06.79 US 53606**

(43) Date of publication of application:
**11.02.81 Bulletin 81/6**

(45) Publication of the grant of the patent:
**14.12.83 Bulletin 83/50**

(84) Designated Contracting States:
**BE CH DE FR GB IT LI LU NL SE**

(56) References cited:
**DE - C - 1 059 457**
**GB - A - 1 194 106**
**US - A - 2 843 600**
**US - A - 3 317 516**
**US - A - 3 812 148**

**The file contains technical information submitted after the application was filed and not included in this specification**

(73) Proprietor: **THE STANDARD OIL COMPANY**
**Midland Building**
**Cleveland, Ohio 44115 (US)**

(72) Inventor: **Pesa, Fred Anthony**
**764 Circlewood Drive Aurora**
**Portage County Ohio 44202 (US)**
Inventor: **Graham, Anne Marie**
**6290 Greenwood Parkway No. 103**
**Northfield, Ohio 44067 (US)**

(74) Representative: **Smith, Sydney et al,**
**Elkington and Fife High Holborn House 52/54**
**High Holborn**
**London WC1V 6SH (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 023 751 B1

# Process for producing five, six or seven membered saturated nitrogen containing heterocyclic compounds

This invention relates to a process for producing five, six or seven-membered saturated nitrogen-containing heterocyclic compounds.

There are several known processes for producing the heterocyclic compounds of the type just referred to. The five membered heterocyclic compound, pyrrolidone, can be produced by the hydrogenation of methyl betacyanopropionate (U.S. 2,843,600). This patent discloses that at temperatures above 150°C pyrrolidone is obtained by slowly contacting an alkyl beta-cyanopropionate with a Raney nickel catalyst. Seven membered nitrogen-containing rings, e.g. caprolactam, are normally prepared from phenol via cyclohexanone oxime. The cyclohexanone oxime is converted to caprolactam by the Beckman rearrangement (see Encyclopedia of Chemical Technology, Kirk-Othmer, Vol. 16 (Second Edition 1968)).

The above processes each have disadvantages, firstly because the catalysts used are prone to deactivation, and secondly because the processes must be conducted in the presence of a large amount of $NH_3$ and at very high catalyst concentrations. The process of the present invention has the advantage that it can be conducted continuously at low catalyst to hydrogen ratios, (high hydrocarbon to catalyst ratios) in the absence of ammonia, and furthermore, the catalysts used according to the invention have long active lives and result in high yields and selectivities of the desired product.

In GB—A—1194106 there is described a process for the production of pyrrolidones from nitrile compounds of the formula

$$NC-\underset{|}{\overset{R^1}{}}CH-\overset{R^2}{\underset{|}{}}CH-COOR^3$$

in which $R^1$ and $R^2$ which may be the same or different, represents a hydrogen atom or an aliphatic, alicyclic or aromatic radical and $R^3$ represents an aliphatic radical, wherein the nitrile compound is passed in gaseous form together with hydrogen at a temperature of from 80 to 300°C over a fixed bed carrier catalyst comprising a Raney metal, a metal selected from cobalt, nickel ruthenium, rhodium, palladium, osmium, iridium or platinum or a mixed catalyst thereof with aluminium and/or a solid reaction product of an oxide of a metal selected from cobalt, nickel, ruthenium, rhodium, palladium, osmium, iridium, and platinum or copper oxide with chromium oxide the molar ratio of nitrile compound to hydrogen being from 1:50 to 1:500.

The technical advance of the process according to that invention is said to be embodied in the fact that it may be carried out at atmospheric pressure so that it may readily be carried out in a continuous cycle, leading to yields equal to those obtained by working under pressure.

According to the present invention there is provided a process for producing a five, six or seven-membered saturated nitrogen-containing heterocyclic compound that is to say a pyrrolidone, a piperidone or a caprolactam comprising contacting a beta, gamma or delta-cyanoester with hydrogen in the presence of a catalyst in which the catalyst is one which contains ruthenium and/or iron, under a pressure of 7—140 kg/cm$^2$.

The invention is particularly applicable to the production of five-membered and seven-membered nitrogen containing heterocyclic compounds, that is to say to pyrrolidones and caprolactams. The catalyst may be one comprising an active catalytic metal or one comprising an oxide metal complex as described more fully below.

Thus, in accordance with the present invention, improved yields and selectivities of five, six and seven-membered nitrogen-containing heterocyclic compounds are obtained by hydrogenating a cyanoester in the presence of a catalyst which comprises at least one of ruthenium and iron. The overall reaction taking place in this process is represented by the following equation:

(A)

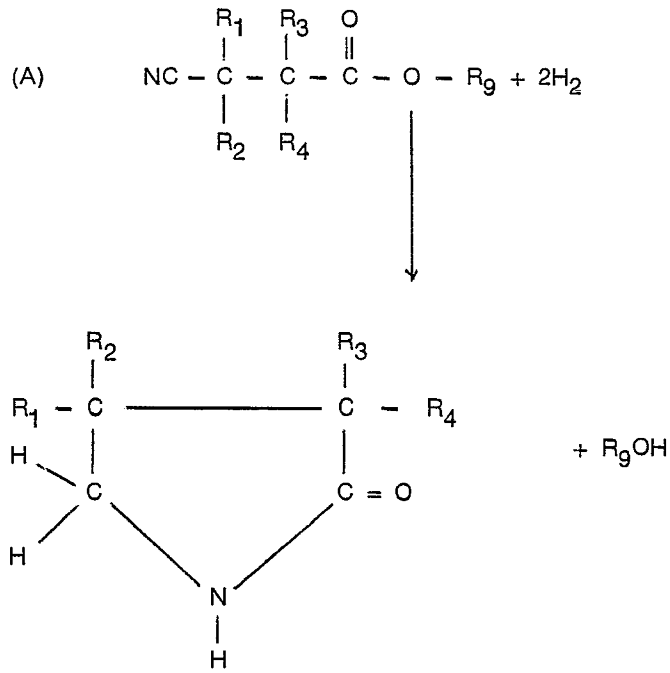

(pyrrolidones)

(B)

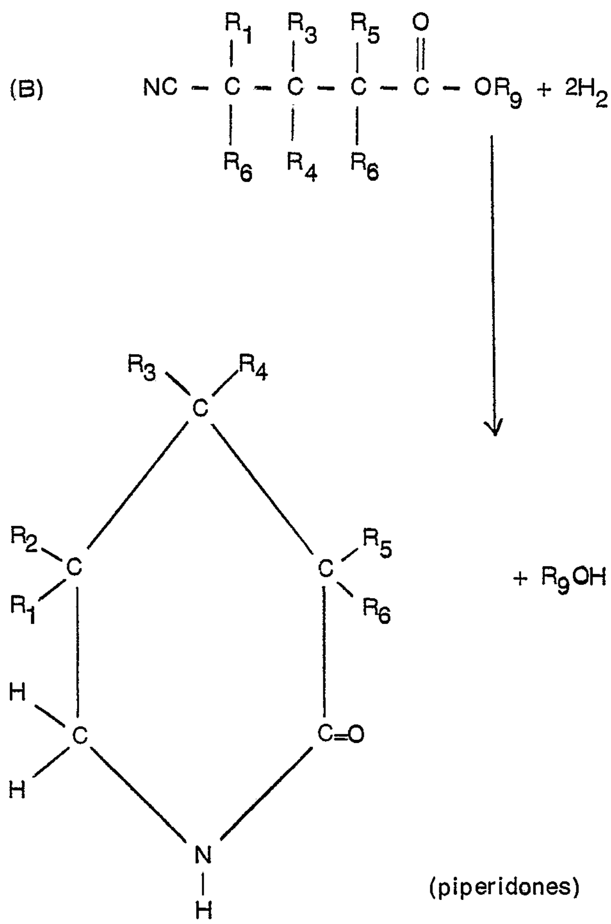

(piperidones)

(C)

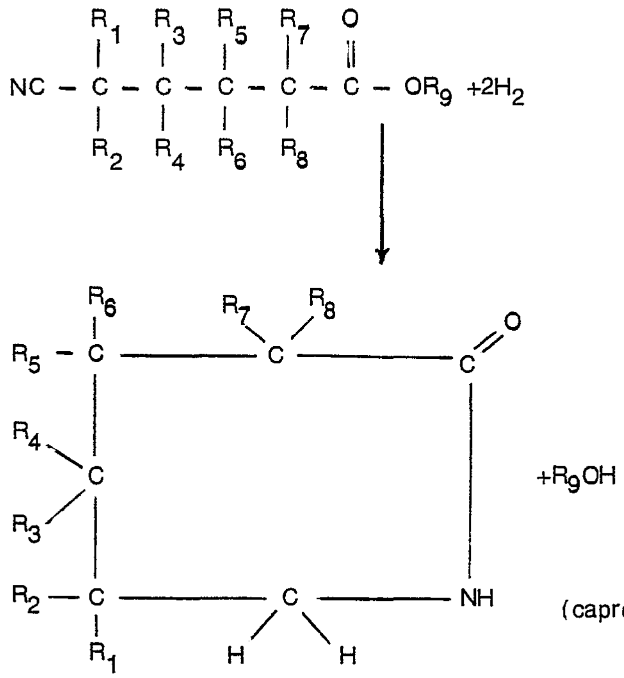

(caprolactams)

wherein $R_1$, $R_2$, $R_3$, $R_4$, $R_5$, $R_6$, $R_7$, $R_8$, and $R_9$ are defined below.

Generally, any beta, gamma or delta-cyanoester can be employed as a reactant in the process according to the invention. However, steric hindrance may become a factor and the reaction rate may be reduced if the cyanoester is substituted with a bulky group.

Preferred cyanoester compounds which may be used in the process of the present invention have the following structure:

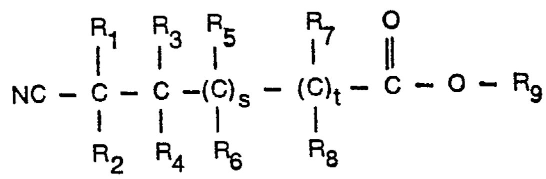

wherein s and t are 0 or 1; and
wherein $R_1$, $R_2$, $R_3$, $R_4$, $R_5$, $R_6$, $R_7$ and $R_8$ are each independently selected from:

(1) hydrogen;
(2) $C_{1-4}$ alkyl;
(3) $-(CH_2)_n-O-(CH_2)_r-H$, wherein n and r are each independently 0—4;
(4) $-(CH_2)_n-\overset{O}{\overset{\|}{C}}-O-(CH_2)_r-H$, wherein n and r are each independently 0—4; and

wherein $R_9$ is selected from:
(1) $C_{1-30}$ alkyls; and
(2) carbocyclic radicals containing up to 30 carbon atoms.

Preferably, the cyanoester are those in which $R_1$, $R_2$, $R_3$, $R_4$, $R_5$, $R_6$, $R_7$, and $R_8$ are each independently selected from:
(1) hydrogen;
(2) methyl;
and wherein $R_9$ is selected from the group consisting of:
(1) $C_{1-4}$ alkyls;
(2) phenyl.

Particularly preferred cyanoesters are those wherein $R_1$, $R_2$, $R_3$, $R_4$, $R_5$, $R_6$, $R_7$, and $R_8$ are each independently selected from hydrogen and wherein $R_9$ is selected from the group consisting of $C_{1-4}$ alkyls.

Since there is already a large commercial market for pyrrolidone, i.e. the five membered ring, and caprolactam, i.e. the seven membered ring, the production of these materials is of particular significance and therefor s and t are preferably both 0 or both 1.

Specific starting materials for use in the process of this invention are methyl-beta-cyanopropionate, 4-cyano-butyrate and 5-cyano-valerate. A process for producing these cyanoesters is disclosed in U.S. 4,238,359.

The amount of hydrogen in the reaction is not particularly critical, since the reaction will proceed if any amount of hydrogen is present. It is preferred however, to use at least two moles of hydrogen per mole of cyanoester. If desired, a carrier gas which is inert to the reactants, products and catalyst can be included in the reaction system.

Furthermore, any material which is inert to the reactants, catalyst and products of this reaction may be included with the cyanoester as a diluent. For example, methanol or ethanol could be added to the reaction system, if desired.

In carrying out the process of the present invention, the cyanoester and hydrogen are contacted in the presence of the catalyst described below to effect the hydrogenation/cyclization reaction. This reaction can be accomplished both in the batch mode and continuously and in either the gas phase or liquid phase.

The reaction temperature is normally maintained between 50°C and 200°C, preferably 75°C to 125°C, and most preferably around 100°C. The reaction pressure is maintained between 100 and 2,000 psi, 7 to 140 $kg/cm^2$ and preferably between 500 to 1,200 psi, 35 to 84 $kg/cm^2$. When the reaction is carried out in the batch mode, the reactants and catalysts are contacted with one another for a period of 10 minutes to 6 hours, and preferably $\frac{1}{2}$ hour to 3 hours. A reaction time of less than 10 minutes or more than 6 hours can be used if desired although better results will be obtained if the reaction time is maintained within this range. When the process is carried out on a continuous basis, the reactant catalyst contact time is normally 10 seconds to 10 minutes, preferably 100 seconds to 5 minutes.

Any catalyst which includes one of ruthenium and/or iron may be employed in the instant process. In particular, both active catalytic metal catalysts and oxide complex catalyst are effective in this invention. Oxide complex catalysts which may be used may be represented by the following general formula:

$$A_aD_bRu_cFe_dO_x$$

wherein A is selected from the group consisting of Co, Ni, Rh, Pd, Os, Ir, Pt and mixtures thereof;
D is selected from the group consisting of Cr, Mo, W, Mn, Re, any mixtures thereof; and
wherein a, b, c and d are 0 to 1; with the proviso that c and d cannot both be equal to 0; and
wherein x represents the number of oxygens required to satisfy the valence requirements of the other elements present in the catalyst.

The oxide complex catalyst can be any catalyst delineated by the general formula above with respect to the components of the catalyst. Preferred catalysts are those wherein A is present (a is greater than 0 and is one or more of Co and Ni and wherein D is one or more of Cr and Re. Especially preferred are those catalysts which contain both Ru and Fe, although one of these may be absent (c = 0 or d = 0). In a preferred embodiment at least 50 mole % of the active catalyst (excluding any support material) component is Ru.

The exact chemical nature of this oxide complex catalyst is not known. This catalyst may be a mixture of oxides, for example, or an oxide complex of all the contained elements. In any event, this type of catalyst is generally known in the art.

The oxide catalyst can be made by techniques which are essentially the same as those techniques described in the art for other oxidation catalysts. (See U.S. 3,642,930). Even though there are numerous techniques that may be utilized to give acceptable oxide complex catalysts, some of the preferred methods of making these catalysts are described below.

The oxide complex catalysts can be prepared from any mixture of compounds that can give the desired oxide components. Preferably, the catalysts are prepared by coprecipitating decomposable salts such as nitrates, acetates, halides and/or oxides. These catalysts are effective in both the calcined and uncalcined form. Other known oxide complex catalyst preparation techniques, however, can be employed.

Active catalytic ruthenium and/or iron metal is also effective in the process of the invention. These catalysts can be prepared by any of the well known techniques available in the art. (See U.S. 3,784,617). For example, the active catalyst metal can be prepared from any material that can be at least partially reduced to give the desired metal.

Preferably, the metallic catalyst is prepared by heat treating and hydrogen reducing a decomposable metal salt, e.g. nitrate, acetate, halide, etc.

Although the above technique for preparing a metallic catalyst is preferred, other preparation techniques are known to those of ordinary skill in the art. These alternate preparation techniques are included in the scope of the instant invention.

Both the oxide complex and active catalytic metal catalysts can be in the supported, unsupported or coated form. Preferred support materials are silica, $ZrO_2$, alumina, phosphates, silica-alumina and zeolites. Any other known support material can be used which is stable under the reaction conditions to be encountered in the use of the catalyst. In the supported form, the support preferably comprises 5%

to 95% by weight of the catalyst, preferably 10% to 60% by weight of the catalyst. In the coated catalyst form the inert core material is preferably in the range of from about 20% to 99% by weight of the catalyst.

The reaction product obtained upon completion of the reaction is normally in the form of a liquid and composed primarily of unreacted reactant and heterocyclic compounds. This reaction product can be subjected to suitable known separation techniques, e.g. solvent extraction or fractional distillation, to yield the desired end product.

The reaction mixture can be made free of catalyst by filtration. The heterocyclic compound can then be separated from the reactant by fractional distillation. It is preferably to conduct this distillation under reduced pressure.

The heterocyclic compounds produced by this process are useful inter alia as precursors to polymers.

The following Examples illustrate the invention. In these examples conversion and yield are defined as follows:

$$\text{Conv.} = \frac{\text{Moles Cyanoester Reacted}}{\text{Moles Cyanoester Fed}} \times 100$$

$$\text{Yield} = \frac{\text{Moles Product}}{\text{Moles Cyanoester Fed}} \times 100$$

The hydrogenation products were analyzed by gas chromatography using a Hewlett-Packard 5750. The examples were performed as follows:

Example 1

Hydrogenation Using Metallic Catalysts

Methyl-3-cyanopropionate was hydrogenated in the presence of a metallic ruthenium catalyst. This catalyst was prepared as follows. 5 grams of $RuCl_3.3H_2O$ and 250 ml. of distilled $H_2O$ were reduced with 6.5 grams of $NaBH_4$ and 250 ml. of $H_2O$ slowly over the course of one hour. The resulting solid was then washed with $H_2O$ and MeOH and then dried in a nitrogen atmosphere. 0.15 grams of this metallic ruthenium catalyst was placed in a 300 ml. Parr autoclave. 1.0 gram of the cyanoester and 49.0 grams of methanol were also placed inside the autoclave. The autoclave was sealed, flushed twice with 200 psi of hydrogen, and then charged to 49 kg/cm$^2$ 700 psi. The autoclave was heated to 100°C and pressure adjustments, if necessary, were made. The reaction was then allowed to proceed for 1 hour. Following this, the autoclave was cooled and vented and the liquid analyzed. A 62% yield of pyrrolidone was obtained.

Experiments 2 and 3

Hydrogenation using Simple Ruthenium and Iron Oxide Catalysts

Example 2

$RuO_2$ Catalyst

An $RuO_2$ catalyst was purchased from Alfa/Ventron of Danvers, Massachusetts. This catalyst was purchased in the form of $RuO_2.H_2O$ and contained 53% ruthenium. 0.5 grams of this catalyst was placed in the experimental apparatus described in Example 1. An 81% yield of pyrrolidone was obtained.

Example 3

$Fe_2O_3$ Catalyst

An $Fe_2O_3$ catalyst was purchased from Matheson, Coleman and Bell. 0.5 grams of this catalyst was placed in the experimental apparatus described in Example 1. The conditions were changed so that the reaction proceeded for 90 minutes at 150°C and 84 kg/cm$^2$ 1,200 psi. An 80% yield of pyrrolidone was obtained.

Examples 4—8

Hydrogenation Using Mixed Oxide Catalysts

Example 4

$RuFeO_x$ Catalyst

Methyl-3-cyanopropionate was hydrogenated to pyrrolidone in the presence of an $RuFeO_x$ catalyst. This catalyst was prepared as follows. 2.70 grams of $FeCl_3.6H_2O$ and 2.53 grams of $RuCl_3.XH_2O$ were dissolved in 150 ml. of distilled water and stirred for 30 minutes. a 50% aqueous solution of NaOH was added dropwise to bring the pH up to 8.3. The resulting slurry was heated near boiling for 30 minutes with constant stirring, and then cooled. The pH was rechecked and found to be 7.4. The mixture was filtered and washed thoroughly and then reslurried in 150 ml. of distilled water. This mixture was again filtered and washed. The black solid obtained was dried overnight at 125°C and

then calcined at 250°C for three hours. Before use, this catalyst was ground to pass 140 mesh.

The catalyst was placed in the experimental apparatus disclosed in Example 1 under the conditions specified in Table 1. A 99% yield of pyrrolidone was obtained.

Example 5

$RuCoO_x$ Catalyst

An $RuCoO_x$ catalyst was also used to hydrogenate Methyl-3-cyanopropionate. The catalyst preparation disclosed in Example 4 was followed except that 2.38 grams of $CoCl_2.6H_2O$ was used instead of 2.70 grams of $FeCl_3.6H_2O$. This catalyst was also placed in the experimental apparatus of Example 1 under the reaction conditions disclosed in Table 1. A 93% yield of pyrrolidone was obtained.

Examples 6—8

$FeDO_x$ Catalysts

Promoted iron oxide catalysts were prepared by the technique shown in Example 4 and placed in the experimental apparatus of Example 1 under the reaction conditions shown in Table 1. The results of these experiments are also shown in Table 1.

Examples 9 and 10

Hydrogenation Using Catalysts Containing Various Ru/Fe Ratios

Example 9

$RuFe_2O_x$ Catalyst

An $RuFe_2O_x$ catalyst was prepared by the catalyst preparation disclosed in Example 4 except that 1.27 grams of $RuCl_3.XH_2O$ was used instead of 2.53 grams of $RuCl_3.XH_2O$. This catalyst was placed into the experimental apparatus disclosed in Example 1 under the reaction conditions specified in Table 1. A 45% yield of pyrrolidone was obtained.

Example 10

$Ru_2FeO_x$ Catalyst

An $Ru_2FeO_x$ catalyst was obtained by the procedure disclosed in Example 4 except that 1.35 grams of $FeCl_3.6H_2O$ was used instead of 2.70 grams of $FeCl_3.6H_2O$. This catalyst was placed into the experimental apparatus of Example 1 under the conditions disclosed in Table 1. An 88% yield of pyrrolidone was obtained.

Table 1

Hydrogenation of Methyl-3-Cyanopropionate in an Autoclave in the Presence of Mixed Oxide Catalysts

Temperature—100°C
Pressure—58.8 kg/cm²
(840 psi)$H_2$

Reaction Time—7 Hours
Ester: Catalyst Ratio
(Molar)—50:1

| Example | Catalyst | Yield of Pyrrolidone (%) |
|---|---|---|
| 4 | $RuFeO_x$ | 99 |
| 5 | $RuCoO_x$ | 93 |
| 6 | $FeNiO_x$ | 34 |
| 7 | $FeCoO_x$ | 33 |
| 8 | $FeRhO_x$ | 65 |
| 9 | $RuFe_2O_x$ | 45 |
| 10 | $Ru_2FeO_x$ | 88 |

Examples 11—14

Hydrogenation Using $RuFeO_x$ Catalyst Under Various Process Conditions

The catalyst prepared in Example 4 was placed in the experimental apparatus of Example 1 under the reaction conditions disclosed in Table 11. The yield of pyrrolidone for each of these examples is also disclosed in Table 11.

Examples 15—16

Hydrogenation Using $RuFeO_x$ Catalyst at Various Catalyst Concentrations

The $RuFeO_x$ catalyst prepared in Example 4 was placed into the experimental apparatus of Example 1 for 2 hours at 100°C and 58.8 kg/cm² (840 psi). The yield of pyrrolidone for each of these examples is disclosed in Table II.

Table II

Hydrogenation of Methyl-3-cyanopropionate in an Autoclave Under Various Process Conditions

Catalyst: $RuFeO_x$

| Example | Ester:Catalyst Ratio (Molar) | Temp. (°C) | Pressure ($kg/cm^2H_2$) | Reaction Time | Pyrrolidone (%) |
|---|---|---|---|---|---|
| 11⁺ | 50:1 | 100 | 58.8(840)⁺⁺ | 7 | 99 |
| 12 | 50:1 | 150 | 58.8(840) | 4 | 96 |
| 13 | 50:1 | 100 | 91(1,300) | 4 | 99 |
| 14 | 50:1 | 100 | 58.8(840) | 7 | 81 |
| 15 | 8.45:1 | 100 | 58.8(840) | 2 | 81 |
| 16 | 1.74:1 | 100 | 58.8(840) | 2 | 93 |

⁺= This catalyst was not calcined.

⁺⁺= psi values.

Examples 17—21

Hydrogenation Using a Supported $RuFeO_x$ Catalyst

A supported $RuFeO_2$ catalyst was prepared as follows. About 10 grams of the dried and calcined $RuFeO_x$ oxide from Example 4 was ground to 170 mesh and slurried in 15 ml. of water. To this was added appropriate amounts of a 40% Nalco silica solution. The solution was kept thoroughly stirred as it was heated to drive off water. When the mixture reached the consistency of toothpaste, it was put in an oven at 125°C for 15 hours. It was then calcined at 325°C for 3 hours. The catalyst was removed from the oven and ground to 10 to 80 mesh particles.

The above catalyst was placed in a continuous flow reactor. The continuous flow reaction system used a 20 cc. fixed-bed reactor with both gas and liquid feed inlets. This reactor was packed with 20 cc. of catalyst. The system was then charged with hydrogen to 70 kg/cm² (1000 psi), and hydrogen was allowed to pass through the catalyst bed while it was heated to 125°C. When the desired temperature was reached, the system was left to equilibrate for ½ hr. The liquid feed, a 2% solution of cyanoester and methanil, was then pumped in at the appropriate rate and the product was trapped in a cooled collection vessel. The outlet gas was passed through a cooled water scrubber to collect any methanol vapor not condensed with the product. The temperature inside the reactor was monitored continuously with a thermocouple placed 2½ inches from the bottom of the catalyst bed. At the completion of the run, the liquid feed was stopped but hydrogen continued to flow for ½ hr. At that point, the gas was turned off, the reactor was cooled and vented, and the liquid product was analyzed. The results are shown in Table III.

Table III
Hydrogenation of Methyl-3-Cyanovalerate in a Continuous Flow Reaction at Various Catalyst Loading Levels

Temperature—125°C

Pressure—70 kg/cm² (1,000 psi)$H_2$

Catalyst—$RuFeO_x$—silica (20 cc.)

| Example | Wt. % Catalyst on Silica | Feed Rate of Ester (cc/Hr) | Yield of Pyrrolidone (%) |
|---|---|---|---|
| 17 | 5 | 17.5 | 65 |
| 18 | 5 | 35.0 | 58 |
| 19 | 30 | 35.0 | 67 |
| 20 | 50 | 35.0 | 74 |
| 21 | 90 | 35.0 | 55 |

Example 22

Hydrogenation of Methyl-5-Cyanovalerate Using $RuFeO_x$ Catalyst

The $RuFeO_x$ catalyst prepared in Example 4 was placed into the autoclave experimental apparatus described in Example 1. 1.0 gram of methyl-5-cyanovalerate was placed in this apparatus and the reaction proceeded for 7 hours at 58.8 kg/cm²(840 psi) and 100°C. The molar ester/catalyst ratio was 50/1. A 35% yield of caprolactam was obtained.

**Claims**

1. A process for producing pyrrolidones, piperidones and caprolactams comprising contacting a beta, gamma or delta-cyanoester with hydrogen in the presence of a catalyst characterised in that the catalyst used contains ruthenium and/or iron at a pressure within the range of 7—140 kg/cm².

2. A process as claimed in claim 1 characterised in that the catalyst comprises an active catalytic metal.

3. A process as claimed in claim 1 characterised in that the catalyst comprises an oxide complex.

4. A process as claimed in claim 3 characterised in that the catalyst is one of the general formula:

$$A_aD_bRu_cFe_dO_x$$

wherein A is selected from the group consisting of Co, Ni, Rh, Pd, Os, Ir, Pt or mixtures thereof; D is selected from the group consisting of Cr, Mo, W, Mn, Re, and mixtures thereof; and

wherein a, b, c and d are each independently 0—1; with the proviso that c and d cannot both be equal to 0; and x represents the number of oxygens required to satisfy the valence requirements of the other elements present in the catalyst.

5. A process as claimed in claim 4 characterised by the use of a catalyst in which d is 0.

6. A process as claimed in claim 4 characterised by the use of a catalyst in which c is 0.

7. A process as claimed in any of claims 4 to 6 characterised by the use of a catalyst in which a is greater than 0.

8. A process as claimed in claim 7 characterised in that A is at least one of Co and Ni.

9. A process as claimed in any of claims 4 to 8 characterised by the use of a catalyst in which b is greater than 0.

10. A process as claimed in claim 9 characterised in that D is at least one of Cr and Re.

11. A process as claimed in claim 4 characterised in that a catalyst is used in which at least 50 mole % of the catalyst (excluding any support material) is Ru.

12. A process as claimed in claim 4 characterised in that a catalyst is used in which c and d are both greater than 0.

13. A process as claimed in any of claims 1 to 12 characterised in that the cyanoester used has the following structure:

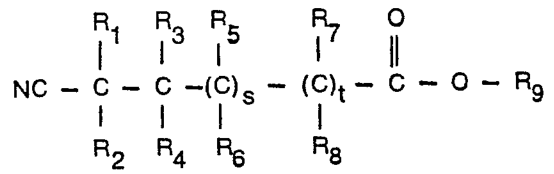

wherein s and t are 0 or 1; and
wherein $R_1$, $R_2$, $R_3$, $R_4$, $R_5$, $R_6$, $R_7$ and $R_8$ are each independently selected from:
(1) hydrogen;
(2) $C_{1-4}$ alkyl;
(3) $-(CH_2)_n-O-(CH_2)_r-H$, wherein n and r are each independently 0—4;
(4)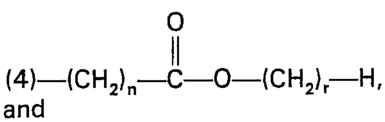, wherein n and r are each independently selected from 0—4; and
wherein $R_9$ is selected from:
(1) $C_{1-30}$ alkyl;
(2) carbocyclic radical containing up to 30 carbon atoms.

14. A process as claimed in claim 13 characterised in that $R_1$, $R_2$, $R_3$, $R_4$, $R_5$, $R_6$, $R_7$ and $R_8$ are each independently selected from hydrogen and methyl, and $R_9$ is $C_{1-4}$ alkyl or phenyl.

15. A process as claimed in any of claims 1 to 14 characterised in that at least two moles of hydrogen are used per mole of cyanoester; and a reaction temperature between 50°C and 200°C is utilized.

**Revendications**

1. Procédé de production de pyrrolidones, pipéridones et carpolactames, comprenant la mise en contact d'un bêta-, gamma- ou delta-cyanoester avec de l'hydrogène en présence d'un catalyseur, caractérisé par le fait qu'on utilise un catalyseur contenant du ruthénium et/ou du fer, à une pression se situant dans la gamme de 6,89—137,89 bars (7—140 kg/cm²).

2. Procédé selon la revendication 1, caractérisé par le fait que le catalyseur comprend un métal catalytique actif.

3. Procédé selon la revendication 1, caractérisé par le fait que le catalyseur comprend un complexe d'oxydes.

4. Procédé selon la revendication 3, caractérisé par le fait que le catalyseur a pour formule générale:

$$A_aD_bRu_cFe_dO_x$$

formule dans laquelle A est choisi dans le groupe formé par Co, Ni, Rh, Pd, Os, Ir, Pt ou leurs mélanges;
D est choisi dans le groupe formé par Cr, Mo, W, Mn, Re et leurs mélanges; et
dans laquelle a, b, c et d sont chacun indépendamment 0—1, à la condition que c et d ne puissent être tout deux égaux à 0; et
x représente le nombre d'oxygènes requis pour satisfaire aux conditions de valence des autres éléments présents dans le catalyseur.

5. Procédé selon la revendication 4, caractérisé par le fait qu'on utilise un catalyseur dans lequel d est 0.

6. Procédé selon la revendication 4, caractérisé par le fait qu'on utilise un catalyseur dans lequel c est 0.

7. Procédé selon l'une des revendications 4 à 6, caractérisé par la fait qu'on utilize un catalyseur dans lequel a est supérieur à 0.

8. Procédé selon la revendication 7, caractérisé par le fait que A est au moins l'une parmi Co et Ni.

9. Procédé selon l'une des revendications 4 à 8, caractérisé par le fait qu'on utilise un catalyseur dans lequel b est supérieur à 0.

10. Procédé selon la revendication 9, caractérisé par le fait que D est au moins l'un parmi Cr et Re.

11. Procédé selon la revendication 4, caractérisé par le fait qu'on utilise un catalyseur dans lequel au moins 50% en mole du catalyseur (à l'exclusion de tout matériau de support) est Ru.

12. Procédé selon la revendication 4, caractérisé par le fait qu'on utilise un catalyseur dans lequel c et d sont tous deux supérieurs à 0.

13. Procédé selon l'une des revendications 1 à 12, caractérisé par le fait qu'on utilise un cyanoester qui présente la structure suivante:

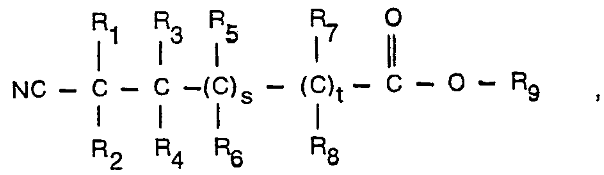

dans laquelle s et t sont chacun indépendamment O ou 1; et dans laquelle $R_1$, $R_2$, $R_3$, $R_4$, $R_5$, $R_6$, $R_7$ et $R_8$ sont choisis chacun indépendamment parmi:
(1) hydrogène;
(2) alkyl en $C_{1-14}$;
(3) $—(CH_2)_n—O—(CH_2)_r—H$, formule dans laquelle n et r sont chacun indépendamment 0—4;
(4) $—(CH_2)_n—\overset{O}{\overset{\|}{C}}—O—(CH_2)_r—H$, formule dans laquelle n et r sont chacun indépendamment choisis dans la gamme 0—4

et dans laquelle $R_9$ est choisi dans le groupe consistant en:
(1) groupe alkyle en $C_{1-30}$;
(2) groupe radical carbocyclique contenant jusqu'à 30 atomes de carbone.

14. Procédé selon la revendication 13, caractérisé par le fait que $R_1$, $R_2$, $R_3$, $R_4$, $R_5$, $R_6$, $R_7$ et $R_8$ sont choisis chacun indépendamment parmi l'hydrogène et le méthyle, et $R_9$ est un alkyle $C_{1-4}$ ou un phényle.

15. Procédé selon l'une des revendications 1 à 14, caractérisé par le fait qu'on utilise au moins deux moles d'hydrogène par mole de cyanoester; et qu'on utilise une température de réaction entre 50°C et 200°C.

**Patentansprüche**

1. Verfahren zur Herstellung von Pyrrolidonen, Piperidonen und Caprolactamen durch Umsetzung durch beta-, gamma- oder delta-Cyanoester mit Wasserstoff in Anwesenheit eines Katalysators, dadurch gekennzeichnet, daß der verwendete Katalysator Ruthenium und/oder Eisen bei einem Druck im Bereich von 7 bis 140 $kg/cm^2$ enthält.

2. Verfahren gemäß Anspruch 1, dadurch gekennzeichnet, daß der Katalysator ein aktives Katalysatormetall enthält.

3. Verfahren gemäß Anspruch 1, dadurch gekennzeichnet, daß der Katalysator einen Oxydkomplex enthält.

4. Verfahren gemäß Anspruch 3, dadurch gekennzeichnet, daß der Katalysator der allgemeinen Formel:

$$A_aD_bRu_cFe_dO_x$$

entspricht, worin A Co, Ni, Rh, Pd, Os, Ir, Pt oder ein Gemisch aus mehreren dieser Elemente ist;
D Cr, Mo, W, Mn, Re oder eine Gemisch aus mehrer dieser Elemente ist; und
worin a, b, c und d jeweils unabhängig voneinander eine Zahl von O bis 1 mit der Maßgabe sind, daß c und d nicht beide die Ziffer O sein können; und
x die Zahl der Sauerstoffatome ist, die notwendig ist um den Wertigkeitsanforderungen der anderen in dem Katalysator anwesenden Elemente zu erfüllen.

5. Verfahren gemäß Anspruch 4, dadurch gekennzeichnet, daß ein Katalysator der angegebenen Formel verwendet wird, worin d Ziffer O ist.

6. Verfahren gemäß Anspruch 4, dadurch gekennzeichnet, daß ein Katalysator der angegebenen Formel verwendet wird, worin c O ist.

7. Verfahren gemäß irgendeinem der Ansprüche 4 bis 6, dadurch gekennzeichnet, daß ein Katalysator der angegebenen Formel verwendet wird, a größer als O ist.

8. Verfahren gemäß Anspruch 7, dadurch gekennzeichnet, daß A mindestens eines der Elemente Co und Ni ist.

9. Verfahren gemäß ingendeinem der Ansprüche 4 bis 8, dadurch gekennzeichnet, daß eine Katalysator der angegebenen Formel verwendet wird, worin e größer als O ist.

10. Verfahren gemäß Anspruch 9, dadurch gekennzeichnet, daß D mindestens eines der Elemente Cr und Re ist.

11. Verfahren gemäß Anspruch 4, dadurch gekennzeichnet, daß ein Katalysator verwendet wird,

worin mindestens 50 Mol% des Katalysators (unter Außerachtlassung von anwesendem Trägermaterial) Ru ist.

12. Verfahren gemäß Anspruch 4, dadurch gekennzeichnet, daß ein Katalysator der angegebenen Formel verwendet wird, worin c und d beide größer als O sind.

13. Verfahren gemäß irgendeinem der Ansprüche 1 bis 12, dadurch gekennzeichnet, daß der eingesetzte Cyanoester der folgenden Formel entspricht:

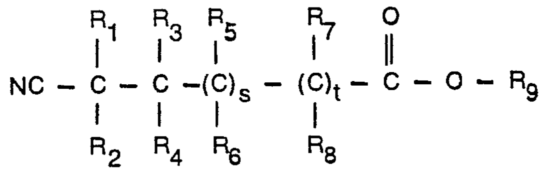

worin s und t unabhängig voneinander eine Zahl O bis 1 sind und worin $R_1$, $R_2$, $R_3$, $R_4$, $R_5$, $R_6$, $R_7$ und $R_8$ jeder unabhängig voneinander aus der Gruppe der folgenden Substituenten gewählt ist:

(1) Wasserstoff;

(2) $C_{1-4}$ Alkyl;

(3) $-(CH_2)_n-O-(CH_2)_r-H$, worin n und r unabhängig voneinander O bis 4 sind;

(4) $-(CH_2)_n-\overset{O}{\overset{\|}{C}}-O-(CH_2)_r-H$, worin n und r unabhängig voneinander O bis 4 sind;

und

worin $R_9$ aus der Gruppe der folgenden Substituenten ausgewählt ist:

(1) $C_{1-30}$ Alkyl;

(2) ein Carbonsäurerest mit bis zu 30 Kohlenstoffatom.

14. Verfahren gemäß Anspruch 13, dadurch gekennzeichnet, daß $R_1$, $R_2$, $R_3$, $R_4$, $R_5$, $R_6$, $R_7$ und $R_8$ unabhängig voneinander Wasserstoff oder Methyl darstellen können und $R_9$ $C_{1-4}$-Alkyl oder Phenyl ist.

15. Verfahren gemäß irgendeinem der Ansprüche 1 bis 14, dadurch gekennzeichnet, daß mindestens 2 Mol Wasserstoff pro Mol Cyanoester verwendet werden und eine Reaktionstemperatur zwischen 50 und 200°C angewandt wird.